# EUROPÄISCHE PATENTSCHRIFT

(11) **EP 1 042 100 B1**
(45) Veröffentlichungstag und Bekanntmachung des Hinweises auf die Patenterteilung: **17.04.2002**
(21) Anmeldenummer: 98965645.9
(22) Anmeldetag: 29.10.1998
(51) Int. Cl.: B23K 26/10, A61F 13/02, A61K 9/70

(54) **LASERSTRAHL-SCHNEIDEVERFAHREN ZUM SCHNEIDEN VON LAMINATEN ZUR APPLIKATION AUF DIE HAUT**
LASER BEAM CUTTING METHOD FOR CUTTING LAMINATES FOR APPLYING TO THE SKIN
PROCEDE POUR DECOUPER AU FAISCEAU LASER DES STRATIFIES S'APPLIQUANT SUR LA PEAU

(30) Priorität: 08.11.1997 DE 19749525
(43) Veröffentlichungstag der Anmeldung: 11.10.2000
(73) Patentinhaber: LTS Lohmann Therapie-Systeme AG, 56626 Andernach (DE)
(72) Erfinder: VON FALKENHAUSEN, Christian, D-53125 Bonn (DE)
(74) Vertreter: Flaccus, Rolf-Dieter, Dr.
(86) Internationale Anmeldenummer: EP9806861
(87) Internationale Veröffentlichungsnummer: WO9924213

(56) Entgegenhaltungen:
- EP-A- 0 418 607
- EP-A- 0 738 556
- WO-A-95/17304
- WO-A-97/11841
- DE-A- 4 110 027
- FR-A- 2 740 714
- US-A- 4 639 572

## Beschreibung

Die Erfindung betrifft ein Verfahren zum Schneiden bzw. eine Vorrichtung für das Schneiden von Laminaten zur Applikation auf die Haut. Diese Laminate weisen mindestens eine Folie und mindestens eine weitere Schicht auf und eignen sich zur medizinisch-pharmazeutischen Applikation auf die Haut.

Die Herstellung von Pflastern aus bahnförmigem Ausgangsmaterial (Laminat) erfolgt üblicherweise - wie etwa gemäß DE-A 41 10 027 - durch mechanisches An- bzw. Durchstanzen der Laminatbahn. Das Stanzmesser besteht dabei aus einem Bandstahlschnitt, welcher beispielsweise in eine Holzschablone eingelegt und fixiert ist. Beim Stanzen wird das Stanzmesser - je nach Laminataufbau mit unterschiedlicher Dynamik - auf die Laminatbahn gefahren und durchstößt die Schichten des Laminats bis zu einer vorbestimmten Stanztiefe. Idealerweise ist dabei der Bandstahlschnitt bezüglich der Laminatbahn exakt horizontal ausgerichtet, so daß das Laminat in allen Punkten längs des Bandstahlschnitts gleichzeitig und gleich tief durchstoßen wird.

Im Produktionsprozeß kommt es allerdings durch die ständige Wiederholung dieser Verfahrensschritte zu einer - je nach Material mehr oder weniger frühen und starken - Abweichung von den vorstehend genannten idealen Bedingungen. Die Gründe hierfür liegen zum einen im Verschleiß des Bandstahlschnitts, zum anderen aber auch in der nicht-idealen Fixierung desselben in der genannten Schablone sowie in weiteren Faktoren, wie etwa dem nicht exakt horizontalen Aufsetzen der Stanze auf das Laminat. Hierdurch kommt es zu einem Anstieg fehlerhafter Stanzvorgänge, so daß die zu durchtrennenden Laminatschichten nicht vollständig durchtrennt werden. Die anschließende Abtrennung des überschüssigen Teil des Laminats, d. h. das Abketten, wird hierdurch erheblich erschwert, woraus ein erhöhter Materialausschuß resultiert. Daher wird ein regelmäßiger Austausch der Bandstahlschnitte nach bestimmten Zeitintervallen erforderlich, was zu erhöhten Kosten infolge der dafür notwendigen Maschinenrüstzeiten und Anfahrverlusten führt. Darüberhinaus unterliegen die Stanzen selbst einem nicht zu vernachlässigenden Verschleiß und bedürfen regelmäßiger Wartung.

Aus WO-A 95/17304 ist ein Verfahren zum Zuschneiden von Etiketten mittels Laserstrahlen, welche auf Pakete appliziert werden, bekannt.

WO-A 97/11841 beschreibt ein Verfahren zur Herstellung von selbstklebenden Etiketten. Diese Etiketten werden mittels eines Schneidekopfs unter Verwendung von Laserstrahlung aus einem bahnförmigen Material geschnitten, wobei das Material unterhalb des Schneidekopfs durch eine Unterdruck-Spannvorrichtung gehalten wird.

Gegenstand der EP 0 418 607 A1 (nächstliegenden Stand der Technik) ist eine Applikationshilfe für flächige Substratabschnitte in Form von Schnitten oder Sollbruchlinien in einem flächenförmigen flexiblen Trägermaterialstreifen. Die Schnitte oder die Sollbruchlinien in der Kontaktfläche zwischen Substrat und Trägermaterial können durch den Einsatz von Laserstrahlen erzeugt werden. Dazu muß der Trägermaterialstreifen einer Schneidezone zugeführt und in dieser gehalten werden.

Aufgabe der Erfindung ist es, ein Verfahren und eine Vorrichtung zum schneiden von Laminaten zur Applikation auf die Haut bereitzustellen, das gegenüber den bekannten derartigen verfahren weniger Werkzeugverschleiß, weniger Materialabfall und insgesamt - u.a. durch die Einsparung von Maschinenrüstzeiten - kostengünstiger ist. Insbesondere soll das Verfahren hochgradig variabel sein, so daß es beispielsweise nicht mehr notwendig ist, für jede gewünschte Form des zu auszustanzenden Laminats eine eigene Stanze zu entwickeln. Diese Aufgabe wird durch ein Verfahren zum Schneiden von Laminaten zur Applikation auf die Haut nach Anspruch 1 bzw. eine vorrichtung nach Anspruch 9 gelöst.

Bevorzugterweise liegt bei dem erfindungsgemäßen Verfahren die Spannung, unter der das Laminat zur Applikation auf die Haut gehalten wird, zwischen einem Wert von 0,1 N/m und 1 x 10⁴ N/m, insbesondere zwischen 600 und 3000 N/m, bevorrugt zwischen 1000 und 2000 N/m. Die Einstellung dieser Spannung kann durch übliche Vorrichtungen, die sich auch außerhalb der Schneidezone befinden können, erfolgen. Zu derartigen erfindungsgemäß verwenteten Vorrichtungen gehören insbesondere vor und/oder hinter der Schneidezone befindliche, im Takt arbeitende zangenartige Vorzüge, welche das Material beispielsweise gegen die bremsende Wirkung einer Schleifkupplung der Laminatspendervorrichtung transportieren oder unterschiedliche Vorzugslängen aufweisen, wobei der in Richtung der Bereitstellung vordere Vorzug einen geringfügig längeren Vorzugsweg aufweist als der hintere.

Gemäß einem weiteren Aspekt der Erfindung wird ein Verfahren zum Schneiden der vorstehend genannten medizinisch-pharmazeutischen Laminate bereitgestellt, das die Schritte umfaßt, daß ein Laminat, umfassend mindestens eine Folie und mipdestens eine weitere Schicht, bereitgestellt wird, das Laminat einer Schneidezone einer Laserstrahlschneideeinrichtung zugeführt; wobei es in der Schneidezone mittels einer Fixiervorrichtung plangehalten wird, sowie mittels Laserstrahlung durch- oder angeschnitten wird.

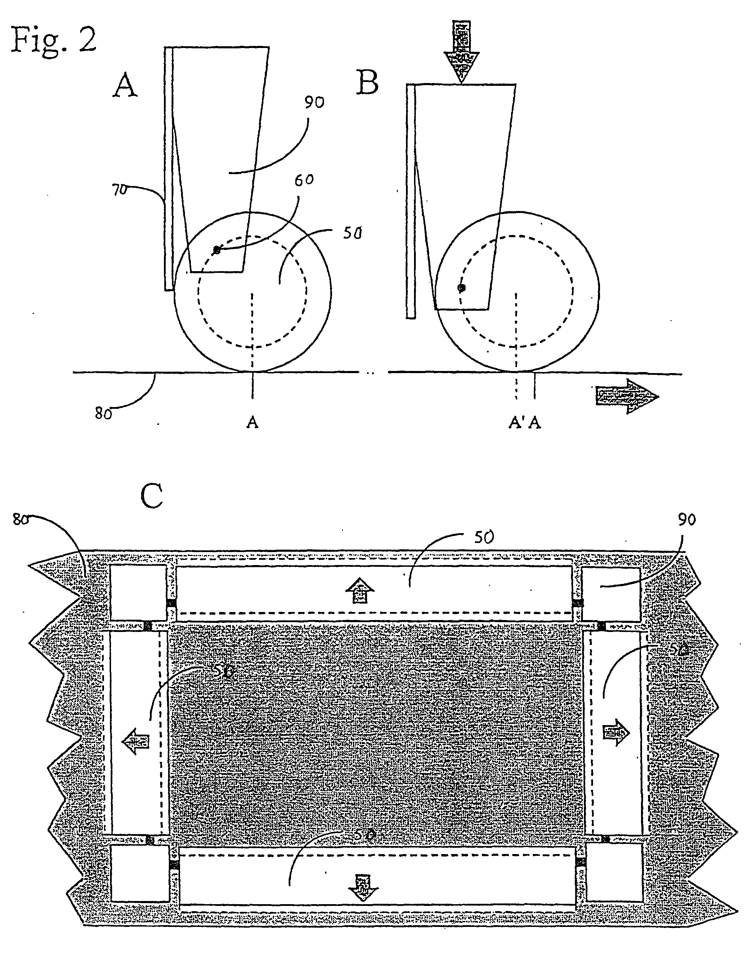

Bei den genannten Fixiervorrichtungen handelt es sich um insbesondere eine wahlweise kugelgelagerte, feststellbare rahmenartige Haltevorrichtung, etwa den nachfolgend erläuterten "aktiven Spannrahmen" (Fig. 2A, B), oder eine an der Oberseite Durchbrechungen aufweisende Haltevorrichtung, wobei der relative Flächenanteil der Durchbrechungen, bezogen auf die Fläche der Oberseite der Fixiervorrichtung, die dem Laserstrahl gegenüberliegt, größer als 50 % ist. Bevorzugt ist dieser Anteil größer als 90 %, insbesondere größer als 99 %. Bevorzugterweise haben die Durchbrechungen eine wabenartige Form.

Andere bevorzugte Fixiervorrichtungen sind eine durchsichtige Abdeckeinrichtung, beispielsweise aus Glas, Kunststoffen wie etwa Plexiglas etc., die eine von dem Laminat abweichende Absorptionsbande aufweisen. Einrichtungen, die auf Kombinationen der vorstehend genannten rahmenartigen Haltevorrichtung, der Durchbrechungen aufweisenden Haltevorrichtung oder der durchsichtigen Abdeckeinrichtung beruhen werden ebenfalls erfindungsgemäß eingesetzt.

Das zur Applikation auf die Haut geeignete Laminat kann erfindungsgemäß während des Schneidevorgangs mit einem Inertgas begast werden. Bei Verwendung bestimmter Materialien können dadurch unerwünschte Oxidationsvorgänge während des Schneidens verhindert werden. Außerdem kann durch das Zuführen von Inertgas während des Schneidens verdampfendes Material weggeblasen oder abgesaugt werden, wodurch Beeinträchtigungen der Laminatoberfläche verhindert werden. Die Beeinträchtigungen können beispielweise darin bestehen, daß verdampfte oder auf andere Art herausgelöste Partikel sich mit der Laminatoberfläche verbinden und deren Eigenschaften in unerwünschter Weise beeinträchtigen. Der vorstehend verwendete Begriff "verdampfen" ist in diesem Zusammenhang weit auszulegen. Darunter ist nicht nur zu verstehen, daß ein festes Material beim Laserschneidevorgang in die Dampfphase übergeht, sondern es sind auch in fester Phase vorliegende Teilchen gemeint, die eine Größe im Bereich von µm oder gar nm oder kleiner aufweisen (nachfolgend auch Mikro- oder Nanopartikel bezeichnet). Unter dem erfindungsgemäß verwendeten Inertgas werden nachfolgend Gase wie Stickstoff, Argon oder Helium usw. verstanden. Die Zufuhr und das Begasen mit dem Inertgas während des Schneidens kann insbesondere dazu verwendet werden, das plane Aufliegen des Laminats auf der erwähnten Fixiervorrichtung zu unterstützen. Dies kann beispielsweise mittels Überdruck erfolgen. Das Gas kann parallel zum Laserstrahl zugeführt und beispielsweise dann mittels Unterdruck abgesaugt werden. Dazu kann man wiederum die erfindungsgemäße Durchbrechungen aufweisende Haltevorrichtung verwenden. Andererseits kann die Zufuhr des Gases auch seitlich, beispielsweise durch Düsen, die sich oberhalb parallel zum Laminat befinden, erfolgen.

Natürlich kann das plane Aufliegen auch dadurch unterstützt werden, daß man an das Laminat unterhalb der Schneidezone Unterdruck anlegt. Dazu eignet sich insbesondere die vorstehend erwähnte Durchbrechungen aufweisende Vorrichtung. Falls das Laminat auf dieser Vorrichtung zum Schneiden aufgelegt ist, kann durch Anlegen eines Unterdrucks an das Laminat vermittels der darunter befindlichen Durchbrechungen das Laminat plangehalten werden.

Unter den erfindungsgemäß verwendeten Laminaten zur Applikation auf die Haut sind prinzipiell alle ein- oder mehrschichtigen Laminate zu verstehen, deren Materialien so beschaffen sind, daß sie sich zum längerfristigen Tragen auf der Haut eignen. Dazu gehören nicht nur die bekannten "Tapes", wie etwa Hansaplast® , Leukosilk® , Leukoplast® etc., sondern auch übliche Pflastermaterialien, insbesondere wirkstoffhaltige Pflaster wie etwa transdermale therapeutische Systeme (TTS). Derartige transdermale therapeutische Systeme sind in K. Heilmann "Therapeutische Systeme - Konzept und Realisation programmierter Arzneiverabreichung" (4. Auflage 1984) beschrieben. Derartige TTS umfassen in der Regel eine Rückschicht, ein bzw. eine ein- oder mehrschichtige(s) Reservoir oder Matrix und eine ggf. abziehbare Schutzschicht in Form einer Folie. Die Rückschicht kann aus flexiblem oder nichtflexiblen Material bestehen. Substanzen, die zu ihrer Herstellung verwendet werden, sind polymere Substanzen, wie etwa Polyethylen, Polypropylen, Polyethylenterephtalat, Polyurethan oder Polyamid. Weitere infragekommende Materialien sind Polyvinylalkohol, Styrol-dien-Blockcopolymere, Polyvinylchlorid, Polymethacrylate, um nur einige weitere Beispiele zu nennen. Natürlich sind auch Kombinationen der erwähnten Materialien einsetzbar. Als weitere Materialien werden auch mit Metall bedampfte Folien wie z. B. mit Aluminium bedampfte Folie, allein oder mit einem polymeren Substrat beschichtet, verwendet. Des weiteren werden auch textile Flächengebilde verwendet, jedenfalls solange die Bestandteile des erwähnten Reservoirs bzw. der Matrix nicht durch sie hindurchdringen können.

Für die wiederentfernbare Schutzfolie können im Prinzip die gleichen Materialien verwendet werden, jedoch muß sie zusätzlich abhäsiv ausgerüstet sein. Diese abhäsive Ausrüstung kann durch eine spezielle Silikonisierung erreicht werden. Das Reservoir bzw. die Matrix, die - wie schon erwähnt - ein- oder mehrschichtig ausgebildet sein können, enthalten in der Regel neben einem oder mehreren Wirkstoffen noch weitere Hiffsstoffe als Zusätze sowie ein Polymermaterial. Infrage kommen dabei Polymere wie Polyisobutylen, Ester des Polyvinylalkohols, Polyacryl- und Polymethacrylsäureester, Naturkautschuk, Styrol-, Isopren- und StyrolButadien-Polymerisate, Siliconpolymere, Harzbestandteile wie gesättigte oder ungesättigte Kohlenwasserstoffharze, Derivate des Abietylalkohols und des β-Pinens, Weichmacher wie Phthalsäureester, Triglyceride und Fettsäuren. Das Polymermaterial der Matrix kann auch auf Polymeren wie Kautschuk, kautschukähnlichen synthetischen Homo-, Co- oder Blockpolymeren, Polyurethanen, Copolymeren des Ethylens oder Polysiloxanen aufgebaut sein.

Die erwähnten Zusätze - auch Hilfsstoffe genannt - werden nach ihrer Furiktion in Weichmacher, Klebrigmacher, Resorptionsvermittler, Stabilisatoren oder Füllstoffe eingeteilt. Derartige Stoffe, die physiologisch unbedenklich sein müssen, sind dem Fachmann grundsätzlich bekannt. Für die beim erfindungsgemäßen Verfahren verwendeten Laminate, insbesondere für die Laminate, aus denen TTS hergestellt werden, können grundsätzlich alle hautgängigen pharmazeutischen Wirkstoffe verwendet werden. Geeignete Wirkstoffe finden sich in den Wirkstoffgruppen der Parasympatholytika (z.B. Scopolamin, Atropin, Benactyzin), der Cholinergika (z. B. Physostigmin, Nicotin), der Neuroleptika (z. B. Chlorpromazin, Haloperidol), der Monoaminoxidasehemmer (z. B. Tranylcypromin, Selegilin), der Sympathomimetika (z. B. Ephedrin, D-Norpseudoephedrin, Salbutamol, Fenfluramin), der Sympatholytika und Antisympathotonika (z. B. Propanolol, Timolol, Bupranolol, Clonidin, Dihydroergotamin, Naphazolin), der Anxiolytika (z. B. Diazepam, Triazolam), der Lokalanästhetika (z. B. Lidocain), der zentralen Analgetika (z. B. Fentanyl, Sufentanil), der Antirheumatika (z. B. Indomethacin, Piroxicam, Lornoxicam), der Koronartherapeutika (z. B. Glyceroltrinitrat, Isosorbiddinitrat), der Östrogene, Gestagene und Androgene, der Antihistaminika (z. B. Diphenhydramin, Clemastin, Terfenadin), der Prostaglandinderivate, der Vitamine (z. B. Vitamin E, Cholecalciferol) und der Cytostatika.

Das Laminat kann der Schneidezone entweder kontinuierlich oder aber getaktet zugeführt werden. Dabei wird die Zufuhrgeschwindigkeit des Laminats - bevorzugt computerunterstützt - geregelt. Ebenso werden natürlich auch alle übrigen Verfahrensparameter des erfindungsgemäßen Laserlaminat-Schneideverfahrens, wie Laserleistung, Geschwindigkeit des Schneidens usw. computerunterstützt geregelt. Im übrigen kann das Laserschneideverfahren naturgemäß sowohl kontinuierlich als auch gepulst durchgeführt werden.

Die Bewegung des Laserstrahls über das Laminat erfolgt dabei insbesondere mittels steuerbarer beweglicher Spiegel, Prismen und/oder Strahlteiler. Insbesondere wird dazu eine Vorrichtung verwendet, welche die den Laserstrahl emittierende Apparatur trägt und in zwei Richtungen (X,Y-Koordinaten) beweglich ausgestaltet ist (Plotterführung): Als Laser selbst kommen alle üblichen Laser infrage. Zu derartigen Lasern gehören Excimerlaser (F₂, ArF, KrF, XeCl, CO, CO₂), Gas-Laser (Ar, HeNe), Festkörper-Laser, Halbleiter-Laser. Der Laserstrahl selbst wird bevorzugt mittels eines Linsensystems auf das Laminat fokusiert.

Bei kontinuierlicher Zufuhr des Laminats werden erfindungsgemäß die Zufuhrgeschwindigkeit und/oder die Bewegung des Lasers dergestalt geregelt, daß die gewählte Schnittform von der Fördergeschwindigkeit unabhängig wird. Die Schnittiefe des Laserstrahls kann dabei konstant oder variabel eingestellt sein. Auf diese Weise kann eine Art "Perforation" erzielt werden. Des weiteren kann aber bei dem erfindungsgemäßen Verfahren auch die Einschnittiefe des Laserstrahls, d. h. bis zu welcher Tiefe das zugeführte Laminat angeschnitten wird, so festgelegt werden, daß unabhängig von einer eventuell vorhandenen Dickenschwankung des Laminats stets immer bis zu einer bestimmten Tiefe angeschnitten wird. Demgemäß kann also die Einschnittiefe in ein mehrschichtiges Laminat längs der Schneiderichtung unterschiedlich sein. Auch wenn das Laminat selbst also keine gleichmäßige Dicke aufweist, sondern mehr oder weniger erhabene Stellen miteinander abwechseln, wie dies bei TTS der Fall sein kann, so wird auf diese Weise erzielt, daß längs der gesamten Anschnittlinie stets bis zu derselben definierten Tiefe angeschnitten wird. Auf diese Weise bleibt die Dicke des verbleibenden Restes des Laminats, der nicht angeschnitten ist, konstant.

Insbesondere kann auch bei dem erfindungsgemäßen Laserschneideverfahren das Intensitätsverteilungsprofil des Laserstrahls angepaßt oder moduliert werden. Dadurch kann die thermische Belastung des Werkstückes gesteuert und so dessen Eigenschaften beeinflußt werden. Beispielsweise kann die Verwendung eines zu den Seiten hin schwach abfallenden Intensitätsprofils, zu dem Vorteil führen, daß in den Randbereichen längs der Schnittkante gezielt thermisch bedingte Veränderungen des zu bearbeitenden Materials herbeigeführt werden. So kann etwa die Viskosität des Materials in dem zu schneidenden Bereich gezielt beeinflußt werden. Bei einem einfachen (Durch-)Schneiden dagegen kann durch Modulierung des Intensitätsprofils die Materialbelastung längs der Schnittkante minimiert werden. Dies wird durch die Verwendung eines möglichst scharfkantigen Intensitätsprofils erreicht. Das zu Erzielung derartiger Schnittkanteneigenschaften erforderliche Intensitätsverteilungsprofil des Laserstrahles hängt jedoch auch von dem verwendeten Laminatmaterial ab und kann nicht vorab allgemeingültig festgelegt werden: Bei einem jeweils vorgegebenen Laminat kann jedoch der Fachmann durch routinemäßige Versuche die zur Erzielung bestimmter Schnittkanteneigenschaften erforderliche Intensitätsverteilung des Laserstrahls festlegen.

Das erfindungsgemäße Verfahren wird durch die nachfolgenden Figuren sowie die Beispiele näher erläutert. Es zeigen die Fig.:

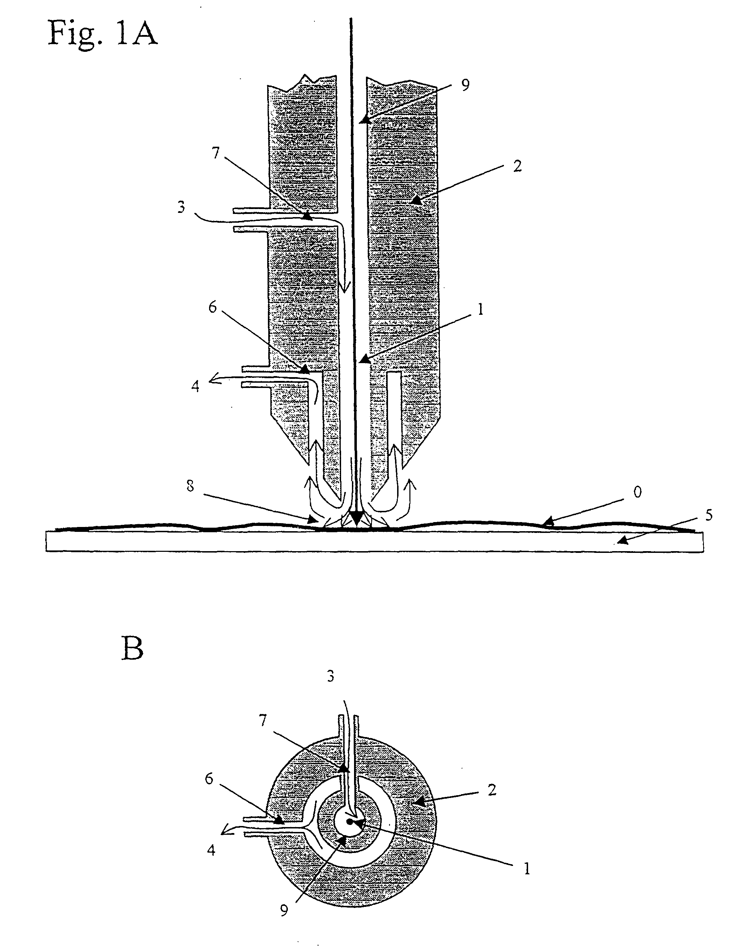

- Fig. 1: eine erfindungsgemäß verwendete Vorrichtung, die das Laminat (0) mittels Überdruck im Bereich der Schneidezone (8) auf der Unterlage (5) plan ausrichtet. Dies erfolgt über einen speziellen Schneidkopf (2) (Fig. 1A Seitenansicht, 1B Aufsicht bzw. Schnitt), welcher über das Laminat geführt wird. Der Schneidkopf umfaßt einen zentralen Kanal (9) durch welchen der Laserstrahl (1) geführt wird. Dieser Kanal kann über eine seitliche Zu- bzw. Abfuhröffnung (7) wahlweise mit Luft oder einem speziellen Prozeßgas (3) geflutet werden. Das Gas strömt unter erhöhtem Druck auf das zu schneidende Laminat (0) und preßt dieses im Bereich der Schneidezone (8) gegen eine Unterlage (5). Hierdurch wird das Laminat in diesem Bereich plan fixiert. Ein weiterer Kanal (6), der ringförmig den zentralen Kanal (9) umfaßt und eine separate Zu- bzw. Abfuhröffnung aufweist, ermöglicht gegebenenfalls das Absaugen beim Schneidevorgang entstehender Partikeldämpfe bzw. Gase (4). Die Öffnungen (6) und (7) können wahlweise als Zuundloder Abfuhröffnung verwendet werden. So kann in einer speziellen Ausführungsform der Vorrichtung über die Öffnung (6) ein weiters Prozeßgas zugeführt werden. Weiterhin kann.Öffnung (7) als Saugkanal fungieren.
- Fig. 2: zeigt im Querschnitt die erfindungsgemäße Fixiervorrichtung in Form eines kugelgelagerten aktiven Spannrahmens in Seitenansicht (oben) (Fig. 2A) bzw. in Aufsicht (unten) (Fig. 2B). Der Spannrahmen weist zwei senkrecht zueinander ausgerichtete Paare exzentrisch gelagerter Rollen (50) auf, wobei die Rollen eines Paares parallel zueinander positioniert sind. Die Lager (90) der Rollen bilden einen rechteckigen Rahmen; die Durchbrechung des Rahmens gibt an ihrer schmalsten Stelle quer zum Laminat (80) mindestens 80% der Laminatbreite frei. Wird das Laminat (80) fixiert bzw. in der Laminatebene gespannt, so bewirkt das (Ab-)Senken des Rahmens, d.h. der Rollen (50), daß durch die exzentrische Lagerung (60) der Rollen (50) das Laminat (80) vom Auflagepunkt A bzw. A' (Fig. 2A, B) in Richtung des Punktes B (Fig. 2B) transportiert wird. Da dies synchron in alle vier Richtungen der Spannebene erfolgt, resultiert hierdurch eine Fixierung des Laminats. Im Ruhezustand der Rollen (50) des Halterahmens verhindert ein Anschlag (70) das Herabhängen der Rollen und sichert dadurch die Richtung des Fördervorganges bei Senkung der Rollen. Eine andere Ausführungsform (nicht gezeigt) des aktiven Spannrahmens umfaßt lediglich ein Paar parallel ausgerichteter Spannrollen, so daß das Spannen des Mateials nur entweder in Laminatförderrichtung oder quer dazu erfolgt.

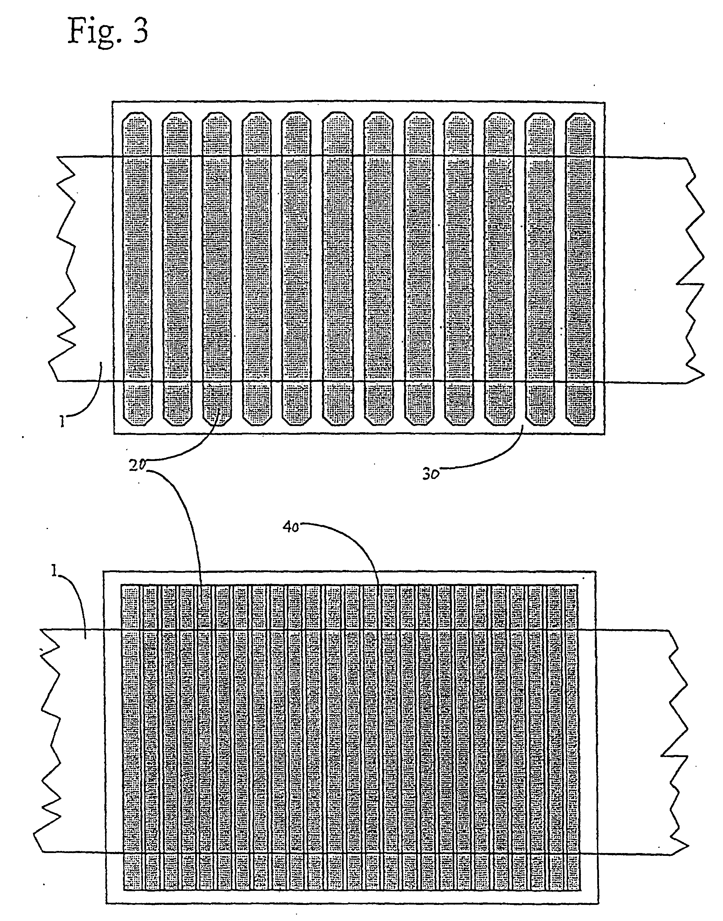

- Fig. 3: zeigt in Aufsicht eine Ausführungsform der Durchbrechungen aufweisende Fixiervorrichtung, nämlich eines Vacuumtischs (30). Die dargestellte Fixiervorrichtung ist derart ausgebildet, daß sie sich besonders zum Durchschneiden des Laminats (1) eignet. Die in der Aufsicht als Ovale erkennbaren Durchbrechungen (20) weisen im Querschnitt an ihrer schmalsten Stelle eine Breite auf; die mindestens dem dreifachen Wert des Durchmessers des Laserstrahls entspricht. Vorzugsweise liegt die Breite der Durchbrechnung zwischen dem dreifachen und dem 100fachen Wert des Durchmessers des Laserstrahls. Der Gesamtanteil der Durchbrechungen an der Fläche der dem Laserstrahl gegenüberliegenden Oberseite liegt bei über 90 %, in der dargestellten ganz besonders bevorzugten Ausführungsform, bei der die Durchbrechungen nur durch schmale Stege (40) aus beispielsweise Metallen wie Aluminium voneinander getrennt sind, bei über 99 %.

## Patentansprüche

1. , Verfahren zum Schneiden von Laminaten zur Applikation auf die Haut, bei dem ein aus mindestens einer Folie und mindestens einer weiteren Schicht gebildetes Laminat (0, 80) bereitgestallt und dieses einer Schneidezone einer Laserstrahlschneideeinrichtung zugeführt und in der Schneidezone plan gehalten und mittels Laserstrahlung durch- oder angeschnitten wird, **dadurch gekennzeichnet, daß** das Laminat (0; 80) zum Planhalten von einer die Schneidezone (8) umgebenden Vorrichtung (2; 50/90) auf eine Unterlage gedrückt und in und quer zur Zuführrichtung gespannt wird.

2. Verfahren nach Anspruch 1, **dadurch gekennzeichnet, daß** das Laminat (0, 80) in der Schneidezone unter einer Spannung zwischen 0,1 N/m und 1 x 10⁴ N/m plan gehalten wird.

3. Verfahren nach Anspruch 2, daß die Spannung einen Wert zwischen 600 und 3000 N/m, bevorzugt zwischen 1000 und 2000 N/m aufweist.

4. Verfahren nach Anspruch 1, **dadurch gekennzeichnet, daß** das Laminat (0) zum Planhalten und Spannen in der Schneidezone (8) durch einen Laserstrahl (1) ungebenden Gasstrom (3) mit Überdruck auf eine Unterlage (5) gedrückt wird.

5. Verfahren nach Anspruch 4, **dadurch gekennzeichnet, daß** der Gasstrom (3) über die Laserstrahleinrichtung (2) zugeführt wird.

6. Verfahren nach einem der vorhergehenden Ansprüche, wobei das während des Schneidevorgangs verdampfende Material entfernt wird.

7. Verfahren nach einem der vorhergehenden Ansprüche, daß während des Schneidens mit Inertgas begast wird.

8. Verfahren nach einem der vorhergehende Ansprüche, wobei das planhalten durch Einsatz von Über- oder Unterdruck unterstützt wird.

9. Vorrichtung für das Schneiden mittels Laserstrahlung von Laminaten zur Applikation auf die Haut, wobei die Vorrichtung eine Fixiervorrichtung aufweist, **dadurch gekennzeichnet, daß** diese Fixiervorrichtung zum Planhalten des Laminates (80) in der Schneidezone einen kugelgelagerten Spannrahmen aufweist, der aus zwei senkrecht zueinander ausgerichteten Paaren exzentrisch an einem Lager (90) gelagerter Rollen (50) gebildet ist, wobei die Rollen (50) eines Paares parallel zueinander ausgerichtet und die exzentrisch angeordneten Drehachsen (60) einander zugewandt sind, daß der Spannrahmen auf das Laminat (80) absenkbar angeordnet ist und die auf das Laminat (80) abgesenkten Rollen (50) das Spannen des Laminates (80) bewirken.

10. Vorrichtung nach Anspruch 9, daß die Fixiervorrichtung im wesentlichen ein thermisch resistentes Material, bevorzugt ausgewählt aus A1, Asbest, Kevlar, Glas, Metallen oder deren Legierungen oder Kunststoffen, enthält.

## Claims

1. Process for cutting laminates intended for application to the skin, wherein a laminate (0; 80) made up of at least one film and at least one further layer is provided and is transported to a cutting area of a laser beam cutting arrangement and is held flat in said cutting area and cut into or cut through by means of laser beam, **characterized in that** to hold the said laminate (0; 80) flat, the same is pressed onto a carrier by a device (2; 50/90) surrounding the cutting area (8), and is tightened in, and transversely to, the direction of advance.

2. Process according to claim 1, **characterized in that** the laminate (0, 80) is kept flat in the cutting area under a tension of between 0.1 N/m and 1 x 10⁴ N/m.

3. Process according to claim 2, **characterized in that** the tension is at a value of between 600 and 3000 N/m, preferably between 1000 and 2000 N/m.

4. Process according to claim 1, **characterized in that** the laminate (0), in order to hold it flat and tighten it in the cutting zone (8), is pressed with excess pressure onto a carrier (5) by means of a gas stream surrounding a laser beam (1).

5. Process according to claim 4, **characterized in that** the gas stream (3) is supplied by means of the laser beam device (2).

6. Process according to one of the preceding claims, wherein the material evaporating during the cutting process is removed.

7. Process according to one of the preceding claims, wherein inert gas is supplied during cutting.

8. Process according to one of the preceding claims, wherein holding the laminate flat is aided by the use of overgressure or underpressure.

9. Device for cutting laminates, which are intended for application to the skin, by means of laser beam, said device having a fixing device, **characterized in that**, to hold the laminate flat in the cutting zone, said fixing device has a ball-bearing tightening frame formed by two pairs of rolls (50), which pairs are arranged perpendicular to each other, and which rolls (50) are eccentrically mounted on a bearing (90), the rolls (50) of a pair being arranged parallel to each other and the eccentrically arranged rotational axes (60) facing each other, and **in that** the tightening frame is arranged such that it can be lowered onto the laminate (80), and the rolls (50) lowered onto the laminate (80) effect the tightening of the laminate (80).

10. Device according to claim 9, **characterized in that** the fixing device essentially contains a thermally resistant material, preferably chosen from aluminum, asbestos, Kevlar, glass, metals and their alloys, or plastics.

## Revendications

1. Procédé pour découper des stratifiés s'appliquant sur la peau, consistant à fournir un stratifié (0 ; 80) constitué par au moins une feuille et au moins une autre couche et à amener celui-ci à une zone de découpage d'un dispositif de découpage au faisceau laser, à le maintenir à plat dans la zone de découpage et à le couper ou l'entamer au faisceau laser, **caractérisé en ce que** le stratifié (0 ; 80), pour être maintenu à plat, est serré contre un support par un dispositif (2 ; 50/90) entourant la zone de découpage (8), et tendu dans le sens d'amenée et perpendiculairement à celui-ci.

2. Procédé selon la revendication 1, **caractérisé en ce que** le stratifié (0 ; 80) est maintenu à plat dans la zone de découpage sous une tension comprise entre 0,1 N/m et 1 x 10⁴ N/m.

3. Procédé selon la revendication 2, **caractérisé en ce que** la tension présente une valeur comprise entre 600 et 3000 N/m, de préférence entre 1000 et 2000 N/m.

4. Procédé selon la revendication 1, **caractérisé en ce que** le stratifié (0), pour être maintenu à plat et tendu dans la zone de découpage (8), est serré contre un support (5) par un courant de gaz (3) en surpression entourant un faisceau laser (1).

5. Procédé selon la revendication 4, **caractérisé en ce que** le courant de gaz (3) est amené par l'intermédiaire du dispositif au faisceau laser (2).

6. Procédé selon l'une des revendications précédentes, dans lequel le matériau s'évaporant pendant le découpage est supprimé.

7. Procédé selon l'une des revendications précédentes, **caractérisé en ce qu'**un gaz inerte est introduit pendant le découpage.

8. Procédé selon l'une des revendications précédentes, dans lequel le maintien à plat est soutenu par application d'une surpression ou d'un vide partiel.

9. Dispositif pour découper au faisceau laser des stratifiés s'appliquant sur la peau, ledit dispositif comprenant un dispositif de fixation, **caractérisé en ce que** ce dispositif de fixation, pour le maintien à plat du stratifié (80) dans la zone de découpage, comprend un cadre tendeur monté sur roulement à billes et qui est constitué par deux paires alignées verticalement l'une sur l'autre de rouleaux (50) montés excentriquement sur un support (90), les rouleaux (50) d'une paire étant alignés parallèlement l'un à l'autre et les axes de révolution (60) agencés excentriquement se faisant face, **en ce que** le cadre de serrage est agencé de manière à pouvoir descendre sur le stratifié (80) et **en ce que** les rouleaux (50) descendus sur le stratifié (80) provoquent la tension du stratifié (80).

10. Dispositif selon la revendication 9, **caractérisé en ce que** le dispositif de fixation contient essentiellement un matériau résistant à la chaleur, choisi de préférence parmi Al, amiante, Kevlar, verre, métaux ou leurs alliages ou matières plastiques.
